# EUROPÄISCHE PATENTSCHRIFT

(11) **EP 2 981 216 B1**
(45) Veröffentlichungstag und Bekanntmachung des Hinweises auf die Patenterteilung: **30.08.2017**
(21) Anmeldenummer: 14725957.6
(22) Anmeldetag: 02.04.2014
(51) Int. Cl.: A61B 17/064

(54) **KLAMMERIMPLANTAT ZUM BEEINFLUSSEN DES WACHSTUMS AN KNOCHENBEREICHEN BENACHBART ZU EINER WACHSTUMSFUGE**
STAPLE IMPLANT FOR INFLUENCING THE GROWTH IN BONE REGIONS BORDERING AN EPIPHYSEAL PLATE
IMPLANT D'AGRAFE DESTINÉ À INFLUENCER LA CROISSANCE SUR DES ZONES OSSEUSES AU VOISINAGE D'UN CARTILAGE DE CROISSANCE

(30) Priorität: 04.04.2013 DE 102013005632; 27.09.2013 DE 102013110759
(43) Veröffentlichungstag der Anmeldung: 10.02.2016
(73) Patentinhaber: Aristotech Industries GmbH, 14943 Luckenwalde (DE)
(72) Erfinder: RÖDL, Robert, 48161 Münster (DE); ANAPLIOTIS, Emmanuel, 14195 Berlin (DE); KAHL, Susanne, 14163 Berlin (DE)
(74) Vertreter: Bittner, Thomas L.
(86) Internationale Anmeldenummer: PCT/DE2014/100115
(87) Internationale Veröffentlichungsnummer: WO 2014/161533

(56) Entgegenhaltungen:
- EP-A1- 0 955 011
- WO-A1-2012/040863
- FR-A1- 2 562 416
- FR-A1- 2 628 312
- FR-A1- 2 934 147
- FR-A1- 2 982 142
- US-A- 4 444 181
- US-A- 4 994 063
- US-A- 5 993 476
- US-A1- 2011 160 766

## Beschreibung

Die Erfindung betrifft ein Klammerimplantat zum Beeinflussen des Wachstums an Knochenbereichen benachbart zu einer Wachstumsfuge.

### Hintergrund

Aus dem Stand der Technik ist eine ganze Reihe von Implantaten bekannt, mit denen versucht wird, Achsfehlstellungen bei Kindern und Jugendlichen vor dem Wachstumsabscltluss zu korrigieren (beispielweise US 8,133,230 B2, US 8,029,507 B2). Diese weisen den Nachteil auf, dass sie aus einer Vielzahl von Teilen zusammengesetzt sind und hohe Fertigungskosten nach sich ziehen. Außerdem tragen die gelenkartig ausgebildeten sperrigen Verbindungsteile und die entsprechenden Schrauben beim Implantieren zu einer vergrößerten Traumatisierung des Patienten bei.

Außerdem sind starre Knochenklammern zur Lenkung des Knochenwachstums an der Epiphysenfuge (Blount-Klammern) bekannt, die zwei parallele Verankerungsschenkel aufweisen, welche durch einen gemeinsamen geradlinigen Quersteg miteinander verbunden sind (EP 0 033 641 20 B1, EPO127994B1, EPO586313B1, EPO852128B1, DE3310833C2, US5,246,443A, US 5,449,359 A, WO 20041107991 A1). Diese Klammern werden vorrangig bei der temporären Epiphyseodese eingesetzt, bei der mindestens zwei Klammern unter Röntgenkontrolle parallel zueinander quer auf die Wachstumsfuge gesetzt werden, deren Wachstum lokal im Klammerbereich blockiert werden soll.

Es sind Klammerimplantate bekannt, bei denen im Bereich eines Querstegs eine Verjüngungszone angeordnet ist, zum Beispiel aus den folgenden Dokumenten: US 2011/160766 A1, US 4,994,063 A, EP 0 955 011 A, US 4,444,181 A, FR 2 628 312 A1 und WO 2012/040863 A1.

Im Dokument US 4,994,063 ist ein Klammerimplantat beschrieben, bei dem beim Einsetzen des Klammerimplantats am Knochen mittels Crimpen mit Hilfe eines Spezialwerkzeugs im Bereich des Querstegs ein Knick hergestellt wird, um Kompressionskräfte auf eine Wachstumsfuge bereitzustellen.

Diese bekannten Klammern vermögen zwar einen wachstumshemmenden Einfluss durch Kompression auf die sich gegenüberliegenden Knochenbereiche auszuüben, lassen aber eine gleichzeitige Aufbiegung des Quersteges der Klammern zur gezielten Förderung des Wachstums an der dem Klammerbereich gegenüberüberliegenden Wachstumsfuge nicht zu.

### Zusammenfassung

Der Erfindung liegt die Aufgabe zugrunde, ein Klammerimplantat bereitzustellen, das eine deformative Verbiegung des Quersteges zum gezielten Öffnen der dem Klammerbereich gegenüberliegenden Wachstumsfuge zur verbesserten Wachstumslenkung unter gleichzeitiger Reduktion der Teileanzahl und vereinfachter Handhabung durch den Chirurgen bei verringerten Kosten ermöglicht.

Diese Aufgabe wird durch ein Klammerimplantat nach Anspruch 1 gelöst. Vorteilhafte Ausgestaltungen sind Gegenstand der Unteransprüche.

Es ist ein Klammerimplantat zum Beeinflussen des Wachstums an Knochenbereichen benachbart zu einer Wachstumsfuge vorgesehen. Das Klammerimplantat weist einen Quersteg und an den Enden des Quersteges angeordnete in Spitzen auslaufende Verankerungsschenkel auf, die zueinander parallel ausgerichtet sind. Die Verankerungsschenkel bilden nach ihrem Einbringen in den Knochen einseitige Fixationspunkte beiderseits der Wachstumsfuge. Der Quersteg weist mindestens eine bei Biegebeanspruchung plastisch deformierbare Verjüngungszone auf, die in beliebiger Lage und Form ohne schroffe Übergänge zwischen den Fixationspunkten ausgebildet ist.

Dies gewährleistet, dass sich die Klammer zwischen ihren Fixationspunkten infolge der an dem Klammerbereich gegenüberliegenden Wachstumsfuge wirkenden Wachstumskräften an der Verjüngungszone aufbiegt und das Wachstum durch Kompression nicht behindert.

Eine Hauptbiegeachse verläuft hierbei vorzugsweise quer zur Verbindungslinie zwischen den Verankerungsschenkel.

Das Klammerimplantat kann aus einem superplastischen biokompatiblen Material bestehen, vorzugsweise Titan, einer Titanlegierung mit nanokristallinen bis ultrafeinkörniger Korngröße (1 nm bis 1 µm). Diese Materialien erreichen eine hohe Festigkeit kombiniert mit einer ausreichend hohen Duktilität bei geringer Ermüdungsneigung.

Die Verjüngungszone kann symmetrisch oder asymmetrisch zur Längsachse im Quersteg gebildet sein. Hierdurch lassen sich die Biegeeigenschaften der Verjüngungszone durch ihre Gestalt und Form an der Ober- und Unterseite des Querstegs beeinflussen, so dass eine gezielte Wachstumslenkung je nach Größe und Umfang der Achsfehlstellung des Patienten erreicht werden kann.

In den Quersteg können Sackbohrungen mit Innengewinde in Flucht der Schenkelachsen eingebracht sein, um ein Einschlaginstruments kraftschlüssig mit dem Quersteg verbinden zu können und das Klammerimplantat an der Wachstumsfuge entsprechend zu setzen.

Alternativ können der Quersteg mit einer umlaufenden Wulst oder Rille zur klemmenden Befestigung an einem Einschlaginstrument und die Sacklöcher mit Innengewinde zum Befestigen eines Extrahierinstruments versehen sein.

Der Biegebereich der Verjüngungszone weist nach seiner Aufbiegung durch die Wachstumskräfte einen Querschnitt auf, der mit einem Trenninstrument vor dem Extrahieren des Implantats leicht durchtrennbar ist.

Der Quersteg und die Verankerungsschenkel können dieselbe oder verschiedene Querschnittsformen aufweisen, beispielsweise Rund-, Viereck- oder Vieleckprofil.

Die Verankerungsschenkel können jeweils mit einem sich in Längsrichtung der Verankerungsschenkel erstreckenden Durchgangskanal gebildet sind. Der Durchgangskanal erstreckt sich vorzugsweise axialsymmetrisch in dem jeweiligen Verankerungsschenkel. Der oder die Durchgangskanäle können beim Implantieren zum Setzen von K-Drähten genutzt werden.

Der Durchgangskanal kann in einem oder beiden Verankerungsschenkeln zumindest abschnittsweise ein Innengewinde aufweist. Hierdurch kann es ermöglicht sein, ein Arbeitswerkzeug in den

Verankerungsschenkel einzuschrauben, zum Beispiel beim beabsichtigten Entfernen des Klammerimplantates.

Die Verankerungsschenkel können auf ihrer äußeren Oberfläche zumindest abschnittsweise Verankerungsmittel mit abstehenden Oberflächenkonturen aufweisen. Die Verankerungsmittel, welche einem unbeabsichtigten Lösen der Verankerungsschenkel nach dem Befestigen des Klammerimplantates am Knochen entgegenwirken, können sich über die gesamte Länge des jeweiligen Verankerungsschenkels erstrecken.

Der Quersteg kann zumindest in einem mittleren Bereich, in welchem wahlweise die Verjüngungszone angeordnet ist, im Querschnitt mit einem Flachprofil gebildet sein. Bei dieser oder anderen Ausführungen kann der Quersteg im Bereich der Verjüngungszone eine Stegdicke von etwa 0,5 bis etwa 3 mm, vorzugsweise von etwa 0,5 bis etwa 1 mm aufweisen.

Der Quersteg kann in einer Ausführung zumindest in dem mittleren Bereich, im Querschnitt mit einem Flachprofil gebildet sein. Bei dieser Ausführung entspricht die Stegdicke der Höhe des Flachprofils. Das Verhältnis von Steghöhe im mittleren Bereich zur Steghöhe an den Enden des Quersteges kann zwischen etwa 3 und etwa 4 betragen, vorzugsweise etwa 3 bis etwa 3,5, insbesondere in Verbindung mit einer einseitigen Anwendung bei einem Klammerimplantat zur Korrektur von X- oder O-Beinen. Besonders bevorzugt ist ein Verhältnis von etwa 3,3. Insbesondere bei einer doppelseitigen Anwendung (medial und lateral, zum Beispiel zum Beinlängenausgleich) kann ein Verhältnis von etwa 0,5 bis etwa 1,5 vorgesehen sein, vorzugsweise von etwa 1. Die Steghöhe im Endbereich kann bei den verschiedenen Ausgestaltungen zwischen etwa 1 und etwa 3 mm liegen, vorzugsweise etwa 2 mm betragen. Im mittleren Bereich mit dem Flachprofil kann die Stegbreite etwa 1,5 bis etwa 3 mm betragen.

Der Quersteg kann in Blickrichtung von oben auf das Klammerimplantat (von Deckseite) zumindest in dem mittleren Bereich gegenüber den Enden des Quersteges verbreitert sein.

Die Verankerungsschenkel können in Blickrichtung von vorn auf das Klammerimplantat zueinander parallel verschoben sein, derart, die Verankerungsschenkel mit dem Quersteg einer Trapezform entsprechend angeordnet sind. Ein die Abweichung von einer rechtwinkligen Anordnung charakterisierender Winkel (Trapezform) kann zwischen > 0° und etwa 25° liegen. Bevorzugt kann ein Winkel von etwa 18° vorgesehen sein.

Die Verankerungsschenkel können auf ihren einander zugewandten Innenseiten eine flache Oberfläche aufweisen, die querstehend zur Verbindungslinie zwischen den Verankerungsschenkeln ausgerichtet ist, vorzugsweise im rechten Winkel hierzu. Die flache Oberfläche kann frei von den Verankerungsmitteln ausgeführt sein.

Die Verjüngung des Quersteges kann mittels einer sich von der Deckseite des Klammerimplantats her erstreckenden Vertiefung gebildet sein. Im Unterschied hierzu kann der Quersteg auf der gegenüberliegenden Innenseite, also nach dem Implantieren dem Knochen zugewandt, eben ausgeführt sein, also insbesondere ohne Vertiefung oder abstehenden Vorsprünge. Hierdurch kann eine ebene Knochenanlagefläche gebildet sein.

### Beschreibung von Ausführungsbeispielen

Im Folgenden werden weitere Ausführungsbeispielen unter Bezugnahme auf Figuren einer Zeichnung näher erläutert. Hierbei zeigen:
- Fig. 1: eine perspektivische Darstellung eines Klammerimplantats mit stirnseitig im Quersteg eingebrachten Sackbohrungen,
- Fig. 2a, 2b: schematische Darstellung der Aufbiegung des Klammerimplantats infolge des Knochenwachstums an der dem geklemmten Bereich gegenüberliegenden Wachstumsfuge,
- Fig. 3: eine Ansicht des Querstegs mit in Flucht seiner Längsachse eingebrachte Ausnehmungen für die 20 klemmenden Befestigung mit einem Einschlaginstrument,
- Fig. 4a bis 4f: Varianten der Lage und Form von Verjüngungszonen im Quersteg des Klammerimplantats,
- Fig. 5: eine schematische perspektivische Darstellung eines weiteren Klammerimplantats mit einer Verjüngungszone im Quersteg,
- Fig. 6: schematische Darstellungen des weiteren Klammerimplantats aus Fig. 5 von vorn und von oben,
- Fig. 7: eine schematische Querschnittsdarstellung des weiteren Klammerimplantats aus Fig. 5,
- Fig. 8: eine schematische Darstellung einer Anordnung mit einem Knochenbereich benachbart zu einer Wachstumsfuge und einem implantierten Klammerimplantat und
- Fig. 9: eine schematische Darstellung der Anordnung aus Fig. 8, wobei nun das Klammerimplantat im Bereich des Quersteges gebogent ist.

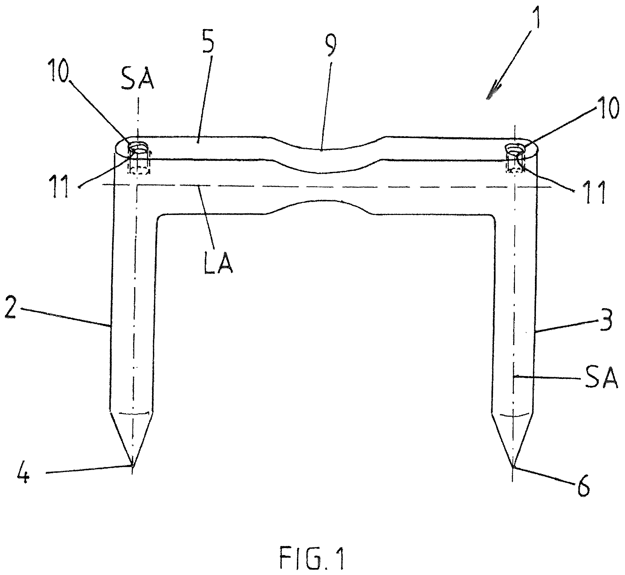

In Fig. 1 zeigt den prinzipiellen Aufbau des einstückigen Klammerimplantats 1, das die Form eines massiven Rundprofiles aufweist, welches beispielsweise aus einem superplastischen biokompatiblen Material besteht. Superplastische Materialien sind nanostrukturierte bzw. ultrafeinkörnige Werkstoffe, die Korngrößen zwischen einigen Nanometern und einem Mikrometer besitzen und ein superplastisches Verhalten, d.h. Dehnungen von mehr als 100% bis sogar 1000% bei hohen Festigkeiten zeigen.

Für das vorliegende Beispiel eines Klammerimplantats haben sich Reintitan oder auch Titanlegierungen als besonders geeignet erwiesen. Es können aber auch andere biokompatible Materialien wie beispielsweise superplastische Stähle eingesetzt werden.

Das Klammerimplantat 1 besitzt zwei Verankerungsschenkel 2 und 3, deren Schenkelachsen SA zueinander parallel ausgerichtet und die an ihren Enden 4 durch einen Quersteg 5 miteinander verbunden sind. Die anderen Enden 6 der Verankerungsschenkel 2 und 3 laufen spitz aus und bilden nach ihrem Einschlagen in die Knochen beiderseits der Wachstumsfuge entsprechende Fixationspunkte 7 und 8.

Der Quersteg 5 besitzt eine Verjüngungszone 9, die symmetrisch zur Längsachse LA des Querstegs 5 verläuft und den mittleren Teil des Querstegs 5 bildet.

In Flucht der Schenkelachse SA sind in den Quersteg 5 Sacklöcher 10 mit einem Innengewinde 11 eingebracht, die jeweils zum Befestigen eines Einschlaginstruments oder eines Extraktionsinstruments dienen. Die Einschlag- oder Extraktionsinstrumente sind handelsüblich und bedürfen daher keiner weiteren Erläuterung.

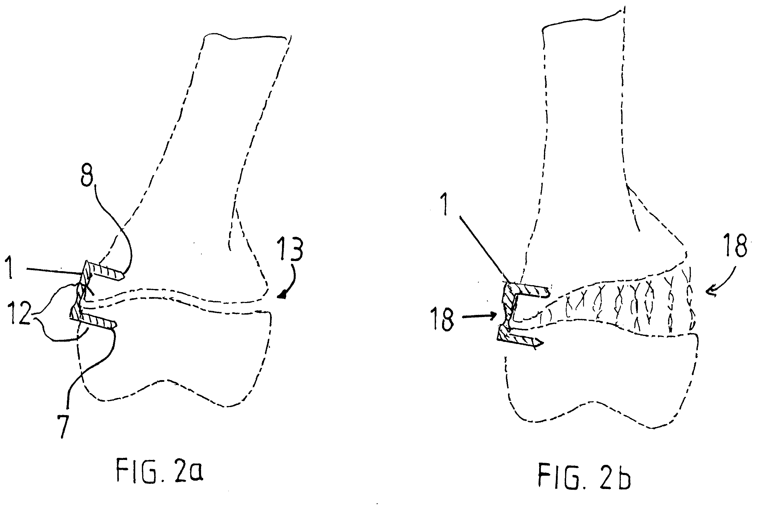

Die Fig. 2a und 2b verdeutlichen die Aufbiegung des erfindungsgemäßen Klammerimplantats 1 in der Verjüngungszone 9, die durch das Knochenwachstum an der dem geklammerten Bereich 12 gegenüberliegenden Wachstumsfuge 13 initiiert wird. Der Biegebereich 18 der Verjüngungszone 9 hat sich bleibend verformt, so dass die Verankerungsschenkel 2 und 3 aufeinander bezogen eine Spreizbewegung ausführen, die zu einer Entlastung der Wachstumsfuge 13 führt und damit das Knochenwachstum nicht behindert.

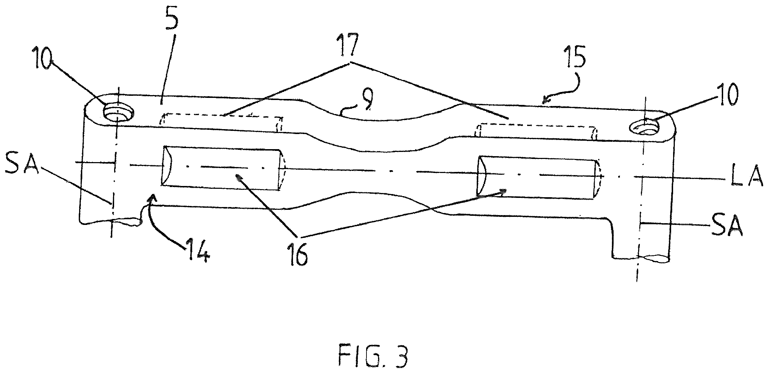

In der Fig. 3 ist beispielsweise eine Ansicht des Querstegs 5 dargestellt, dessen in Flucht der Verankerungsschenkel 2 und 3 verlaufende Seiten 14 und 15 mit jeweils zwei parallel zur Längsachse LA zueinander fluchtenden Aussparungen 16 und 17 versehen sind.

Es gehört aber auch zu der Erfindung, wenn anstelle Aussparungen 16 und 17 entsprechende Wülste an die Seiten 14 und 15 des Querstegs 5 stoffschlüssig angeformt sind.

Die Aussparungen 16 und 17 bzw. Wülste dienen zur Kraftübertragung beim Einschlagen der Schenkel 2 und 3 mit einem üblichen Einschlaginstrument, welches mit seinen Greifelementen in die Aussparungen 16 und des Querstegs 5 kraftübertragend beidseitig eingreift oder die Wülste umgreift und das Klammerimplantat 1 zum Einschlagen in Position bringt. In einem solchen Fall werden die Sacklöcher 10 mit ihren Innengewinden 11 lediglich zur Befestigung des Extraktionsinstrumentes genutzt, wodurch die Innengewinde 11 keiner Schlagbeanspruchung ausgesetzt sind.

Der Biegebereich 18 der Verjüngungszone 9 hat nach seiner Aufbiegung einen solchen Querschnitt, der leicht durch ein Trenninstrument durchtrennbar ist, so dass jeder Verankerungsschenkel 2 bzw. 3 einzeln durch das Extraktionsinstrument schonend entfernt werden kann.

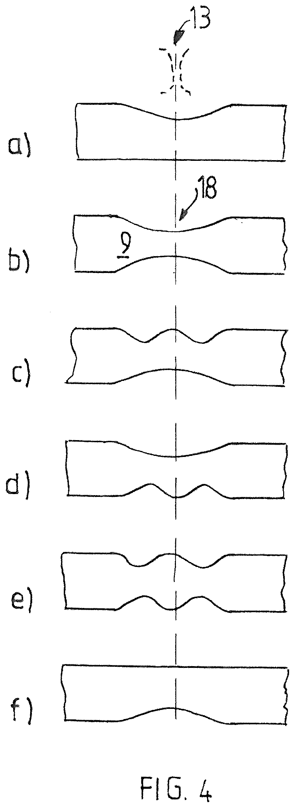

Die Fig. 4a bis 4f zeigen verschiedene Formen der Verjüngungszone 9 im Quersteg 5 des Klammerimplantats 1. Es kann dabei eine symmetrische oder asymmetrische Anordnung mindestens einer Verjüngungszone 9 vorgesehen sein, wobei lediglich scharfe Kanten bzw. Ecken in der Gestalt der Verjüngungszone 9 vermieden werden sollten, um eine Spannungskonzentration an den Kanten oder Ecken 5 vorzubeugen. Der Biegebereich 18 der Verjüngungszone 9 lässt sich demjenigen Querschnitt zuordnen, der innerhalb der Verjüngungszone ein Minimum annimmt.

Das Klammerimplantat 1 kann in verschiedenen Profilformen, beispielsweise als Rund-, Viereck- oder Vieleckprofil, ausgeführt sein.

Das Klammerimplantat hat in seinen verschiedenen Gestaltungen den besonderen Vorteil, dass durch die superplastischen Eigenschaften des Klammermaterials in Verbindung der besonderen Form und Lage der Verjüngungszone eine Kompressionswirkung an der dem geklammerten Bereich 12 gegenüberliegenden Wachstumsfuge 13 ausgeschlossen wird. Das Klammerimplantat 1 ist außerdem sehr einfach im Aufbau und für einen minimalinvasiven Eingriff zur Korrektur einer Achsfehlstellung durch den Chirurgen außerordentlich gut geeignet.

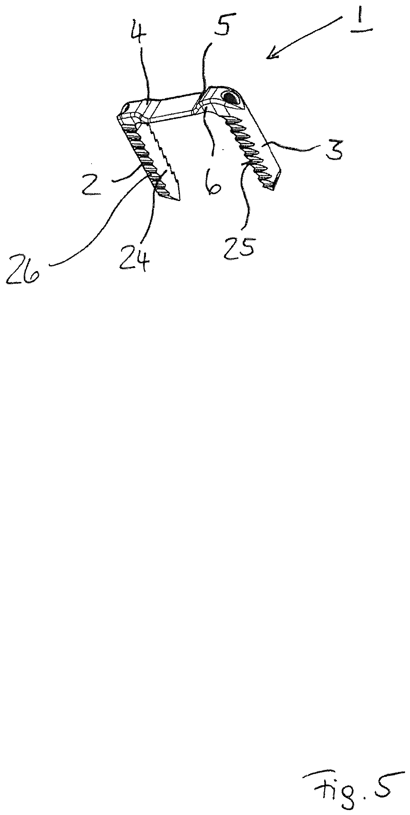

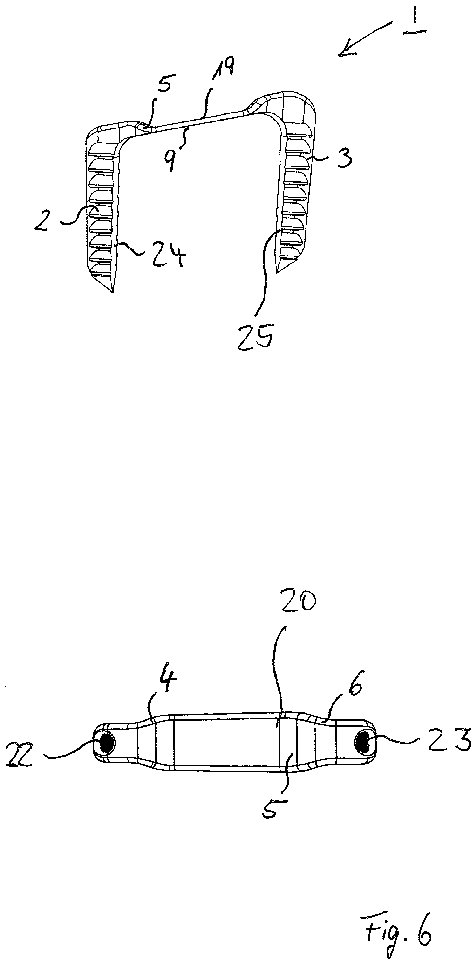

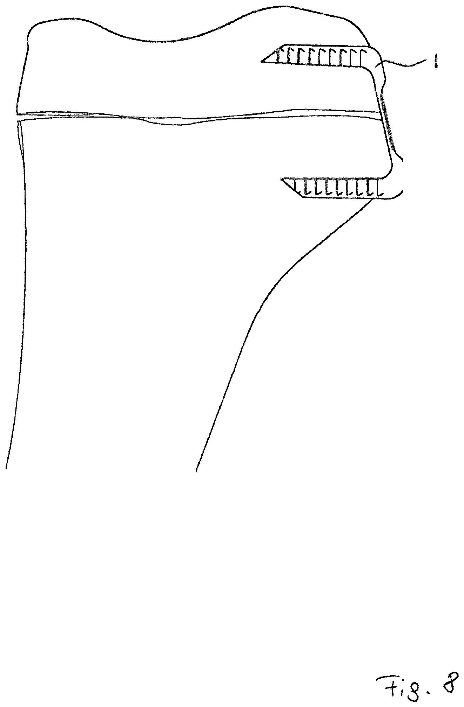

Nachfolgend wird unter Bezugnahme auf die Fig. 5 bis 9 eine weitere Ausführungsform für das Klammerimplantat 1 erläutert. Für gleiche Merkmale werden in den Fig. 5 bis 9 dieselben Bezugszeichen wie in den Fig. 1 bis 4 verwendet.

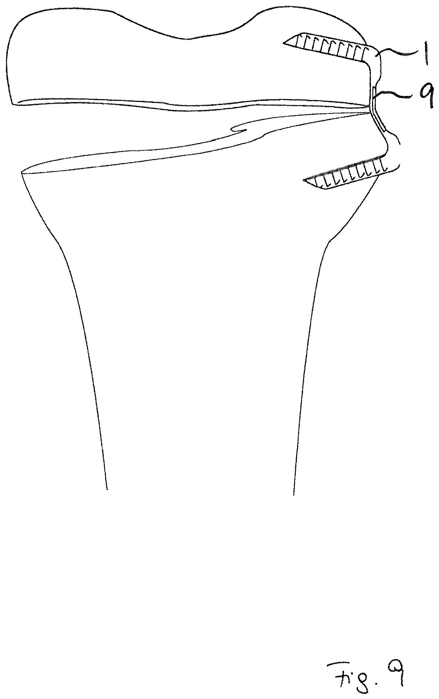

Bei dem Klammerimplantat 1 in Fig. 5 sind ebenfalls die beiden Verankerungsschenkel 2, 3 über den Quersteg 5 miteinander verbunden. Der Quersteg 5 weist die Verjüngungszone 9 auf, die bei der dargestellten Ausführungsform mittig und symmetrisch zur Mitte des Quersteges 5 angeordnet ist mittels einer Vertiefung 19, die von einer Deckseite 20 des Klammerimplantats 1 ausgeht. Im zentralen Bereich 21 der Vertiefung 19 ist der Quersteg 5 mit gleichbleibender Steghöhe als Flachprofil ausgebildet. Zu den Enden 4, 6 des Querstegs 5 hin nimmt die Höhe des Querstegs 5 dann wieder zu. Mit der Verjüngungszone 9 ist ein Biegebereich gebildet, derart, dass sich das Klammerimplantat 1 bei der Verwendung im Zusammenhang mit einer Wachstumsfuge im Bereich des Querstegs 5 verbiegen kann, was insbesondere in Fig. 9 gezeigt ist.

Durch die Verankerungsschenkel 2, 3 hindurch erstreckt sich in axialer Richtung jeweils ein Durchgangskanal 22, 23, welcher für einen oder beide Verankerungsschenkel 2, 3 wenigstens abschnittsweise mit einem Innengewinde versehen sein kann.

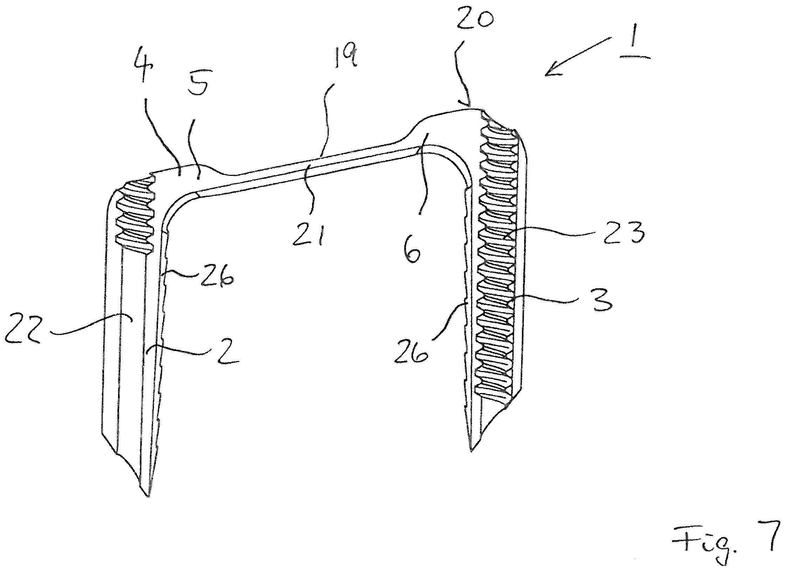

Bei der weiteren Ausführungsform des Klammerimplantats 1 in den Fig. 4 bis 9 sind die beiden Verankerungsschenkel 2, 3 parallel zueinander verschoben, derart, dass sich eine Trapezform ergibt, was insbesondere auch aus Fig. 7 ersichtlich ist.

In Blickrichtung von oben ist der Quersteg 5 bei dem weiteren Klammerimplantat 1 im mittleren Bereich 21 mit größerer Stegbreite ausgebildet als an den Enden 4, 6, wobei sich die Verbreiterung des mittleren Bereiches stetig zu den Enden 4, 6 hin vermindert.

Wie sich insbesondere aus Fig. 5 ergibt, verfügen die Verankerungsschenkel 2, 3 über flache Innenseiten 24, 25, die im Unterschied zu anderen Oberflächenbereichen der beiden Verankerungsschenkel 2, 3 nicht über Verankerungsmittel 26 verfügen. Die Verankerungsmittel 26, die bei der dargestellten Ausführungsform über die Länge der Verankerungsschenkel 2, 3 ausgebildet sind, dienen der Verankerung des Klammerimplantats 1 nach dem Implantieren. Zum gezielten Entfernen des Klammerimplantats 1 kann ein Werkzeug in das Innengewinde der Durchgangskanäle eingeschraubt werden.

Die in der vorstehenden Beschreibung, den Ansprüchen und der Zeichnung offenbarten Merkmale können sowohl einzeln als auch in beliebiger Kombination für die Verwirklichung der verschiedenen Ausführungen von Bedeutung sein.

## Patentansprüche

1. Klammerimplantat zum Beeinflussen des Wachstums an Knochenbereichen benachbart zu einer Wachstumsfuge, mit
- einem Quersteg (5) und
- an den Enden des Quersteges (5) angeordneten, in Spitzen auslaufenden Verankerungsschenkeln (2, 3), die zueinander parallel ausgerichtet sind, wobei die Verankerungsschenkel (2, 3) nach ihrem Einbringen in den Knochen einseitige Fixationspunkte (7, 8) beiderseits der Wachstumsfuge (13) bilden,
wobei der Quersteg (5) mindestens eine bei Biegebeanspruchung plastisch deformierbare Verjüngungszone (9) aufweist, die in beliebiger Lage und Form ohne schroffe Übergänge zwischen den Fixationspunkten (7, 8) ausgebildet ist, **dadurch gekennzeichnet, dass** die Verjüngungszone (9) einen Biegebereich (18) aufweist, welcher sich aufgrund einer Spreizbewegung der Verankerungsschenkel (2, 3) infolge von Knochenwachstum an der dem geklammerten Bereich gegenüberliegenden Wachstumsfuge (13) bleibend verformt.

2. Klammerimplantat nach Anspruch 1, bestehend aus einem superplastischen Material mit Korngrößen zwischen 1 nm und 1 µm.

3. Klammerimplantat nach Anspruch 1 oder 2, **dadurch gekennzeichnet, dass** die mindestens eine Verjüngungszone (9) im Quersteg (5) mittig und ihr Biegebereich (18) weitgehend symmetrisch hierzu angeordnet ist.

4. Klammerimplantat nach mindestens einem der vorangehenden Ansprüche, **dadurch gekennzeichnet, dass** die mindestens eine Verjüngungszone (9) symmetrisch zur Längsachse (LA) im Quersteg (5) gebildet ist.

5. Klammerimplantat nach mindestens einem der vorangehenden Ansprüche, **dadurch gekennzeichnet, dass** Sackbohrungen (10) mit Innengewinde (11) in Flucht der Schenkelachsen (SA) zur kraftschlüssig lösbaren Befestigung eines Einschlag- oder Extraktionsinstrumentes im Quersteg (5) eingebracht sind.

6. Klammerimplantat nach mindestens einem der vorangehenden Ansprüche, **dadurch gekennzeichnet, dass** der Quersteg (5) mit Aussparungen (16, 17) oder Wülsten für eine klemmende Befestigung eines Einschlaginstruments versehen ist.

7. Klammerimplantat nach mindestens einem der vorangehenden Ansprüche, **dadurch gekennzeichnet, dass** der Biegebereich (18) der mindestens einen Verjüngungszone (9) einen Querschnitt aufweist, der mit einem Trenninstrument leicht durchtrennbar ist.

8. Klammerimplantat nach mindestens einem der vorangehenden Ansprüche, **dadurch gekennzeichnet, dass** der Quersteg (5) und die Verankerungsschenkel (2, 3) mit verschiedenen Querschnittsformen ausgeführt sind.

9. Klammerimplantat nach mindestens einem der vorangehenden Ansprüche, **dadurch gekennzeichnet, dass** die Verankerungsschenkel (2, 3) jeweils mit einem sich in Längsrichtung der Verankerungsschenkel (2, 3) erstreckenden Durchgangskanal gebildet sind.

10. Klammerimplantat nach Anspruch 9, **dadurch gekennzeichnet, dass** der Durchgangskanal in einem oder beiden Verankerungsschenkeln (2, 3) zumindest abschnittsweise ein Innengewinde aufweist.

11. Klammerimplantat nach mindestens einem der vorangehenden Ansprüche, **dadurch gekennzeichnet, dass** die Verankerungsschenkel (2, 3) auf ihrer äußeren Oberfläche zumindest abschnittsweise Verankerungsmittel mit abstehenden Oberflächenkonturen aufweisen.

12. Klammerimplantat nach mindestens einem der vorangehenden Ansprüche, **dadurch gekennzeichnet, dass** der Quersteg (5) zumindest in einem mittleren Bereich im Querschnitt mit einem Flachprofil gebildet ist.

13. Klammerimplantat nach mindestens einem der vorangehenden Ansprüche, **dadurch gekennzeichnet, dass** der Quersteg (5) in Blickrichtung von oben zumindest in dem mittleren Bereich gegenüber den Enden des Quersteges (5) verbreitert ist.

14. Klammerimplantat nach mindestens einem der vorangehenden Ansprüche, **dadurch gekennzeichnet, dass** die Verankerungsschenkel (2, 3) in Blickrichtung von vorn zueinander parallel verschoben sind, derart, die Verankerungsschenkel (2, 3) mit dem Quersteg (5) einer Trapezform entsprechend angeordnet sind.

## Claims

1. A staple implant for influencing the growth in bone regions bordering an epiphyseal plate, said staple implant comprising
- a transverse web (5) and
- anchoring limbs (2, 3) provided at the ends of the transverse web (5), oriented parallel to each other and terminating in points, wherein the anchoring limbs (2, 3), after they have been inserted into the bone, form fixation points (7, 8) on one side of said bone, to both sides of the epiphyseal plate (13),
wherein the transverse web (5) comprises at least one tapered zone (9), which is plastically deformable under a flexural load and which is configured in any desired position and shape without abrupt transitions between the fixation points (7, 8), **characterized in that** the tapered zone (9) comprises a bending area (18) which deforms plastically because of an expansion movement of anchoring limbs (2, 3) due to bone growth on the epiphyseal plate (13) lying opposite the stapled area (12).

2. The staple implant as claimed in claim 1, made of a superplastic material having grain sizes of between 1 nm and 1 µm.

3. The staple implant as claimed in claim 1 or 2, wherein the at least one tapered zone (9) is provided centrally in the transverse web (5), and its bending area (18) is provided substantially symmetrically thereto.

4. The staple implant as claimed in at least one of the preceding claims, wherein the at least one tapered zone (9) is provided on the transverse web (5) symmetrically with respect to the longitudinal axis (LA).

5. The staple implant as claimed in at least one of the preceding claims, wherein blind bores (10) with internal threading (11) are provided, in alignment with the limb axes (SA), in order to allow an impaction or extraction instrument to be secured releasably with a force-fit connection in the transverse web (5).

6. The staple implant as claimed in at least one of the preceding claims, wherein the transverse web (5) is provided with recesses (16, 17) or beads for securing an impaction instrument by clamping.

7. The staple implant as claimed in at least one of the preceding claims, wherein the bending area (18) of the at least one tapered zone (9) is provided with a cross section that can be easily separated with a separating instrument.

8. The staple implant as claimed in at least one of the preceding claims, wherein the transverse web (5) and the anchoring limbs (2, 3) are provided with different cross-sectional shapes.

9. The staple implant as claimed in at least one of the preceding claims, wherein the anchoring limbs (2, 3) are each provided with a through-channel extending in the longitudinal direction of the anchoring limbs (2, 3).

10. The staple implant as claimed in claim 9, wherein the through-channel, in one or both anchoring limbs (2, 3), has an internal thread at least in part.

11. The staple implant as claimed in at least one of the preceding claims, wherein the anchoring limbs (2, 3), at least on parts of their outer surface, are provided with anchoring means with protruding surface contours.

12. The staple implant as claimed in at least one of the preceding claims, wherein the transverse web (5) is formed, at least in a central area, with a flat profile in cross section.

13. The staple implant as claimed in at least one of the preceding claims, wherein the transverse web (5), viewed from above, is wider at least in the central area compared to the ends of the transverse web (5).

14. The staple implant as claimed in at least one of the preceding claims, wherein the anchoring limbs (2, 3), viewed from the front, are offset parallel to each other in such a way that the anchoring limbs (2, 3) are arranged with the transverse web (5) in a trapezoid shape.

## Revendications

1. Implant à agrafe permettant d'agir sur la croissance au niveau de zones osseuses à proximité d'une structure de croissance, comportant
- une traverse (5) et
- des branches d'ancrage (2, 3) disposées aux extrémités de la traverse (5) et se terminant en pointe, qui sont orientées parallèlement l'une à l'autre, les branches d'ancrage (2, 3) constituant, après leur introduction dans l'os, des points de fixation unilatéraux (7, 8) des deux côtés de la structure de croissance (13),
la traverse (5) présentant au moins une zone de rétrécissement (9) plastiquement déformable en cas de sollicitation à la flexion et qui est constituée à une position et en une forme quelconque sans transition abrupte entre les points de fixation (7, 8), **caractérisé en ce que** la zone de rétrécissement (9) présente une zone de flexion (18) qui se déforme en raison d'un mouvement d'écartement des branches d'ancrage (2, 3) suite à la croissance osseuse en restant au niveau de la structure de croissance (13) opposée à la zone agrafée.

2. Implant à agrafe selon la revendication 1, composé d'un matériau hyper-plastique ayant une granulométrie de 1 nm à 1 µm.

3. Implant à agrafe selon la revendication 1 ou 2, **caractérisé en ce que** l'au moins une zone de rétrécissement (9) est disposée au centre de la traverse (5) et que sa zone de flexion (18) est dans une large mesure disposée symétriquement par rapport à celle-ci.

4. Implant à agrafe selon au moins une des revendications précédentes, **caractérisé en ce que** l'au moins une zone de rétrécissement (9) est constituée symétriquement par rapport à l'axe longitudinal (LA) dans la traverse (5).

5. Implant à agrafe selon au moins une des revendications précédentes, **caractérisé en ce que** des trous borgnes (10) à filetage intérieur (11) sont pratiqués dans l'alignement des axes des branches (SA) pour la fixation dissociable par correspondance mécanique d'un instrument de percussion ou d'extraction dans la traverse (5).

6. Implant à agrafe selon au moins une des revendications précédentes, **caractérisé en ce que** la traverse (5) est pourvue d'échancrures (16, 17) ou de bourrelets pour une fixation serrée d'un instrument de percussion.

7. Implant à agrafe selon au moins une des revendications précédentes, **caractérisé en ce que** la zone de flexion (18) de l'au moins une zone de rétrécissement (9) présente une section transversale qui peut être facilement coupée avec un instrument de coupe.

8. Implant à agrafe selon au moins une des revendications précédentes, **caractérisé en ce que** la traverse (5) et les branches d'ancrage (2, 3) sont réalisées avec différentes formes de section transversale.

9. Implant à agrafe selon au moins une des revendications précédentes, **caractérisé en ce que** les branches d'ancrage (2, 3) sont respectivement constituées par le canal traversant s'étendant dans le sens longitudinal des branches d'ancrage (2, 3).

10. Implant à agrafe selon la revendication 9, **caractérisé en ce que** le canal traversant présente, du moins par endroits, un filetage intérieur dans une branche d'ancrage ou les deux (2, 3).

11. Implant à agrafe selon au moins une des revendications précédentes, **caractérisé en ce que** les branches d'ancrage (2, 3) présentent sur leur surface extérieure, du moins par endroits, des moyens d'ancrage dotés de contours surfaciques proéminents.

12. Implant à agrafe selon au moins une des revendications précédentes, **caractérisé en ce que** la traverse (5) est réalisée avec un profil plat, du moins dans une partie centrale de sa section transversale.

13. Implant à agrafe selon au moins une des revendications précédentes, **caractérisé en ce que** la traverse (5), dans le sens de visualisation par le haut, est élargie du moins dans sa zone centrale par rapport aux extrémités de la traverse (5).

14. Implant à agrafe selon au moins une des revendications précédentes, **caractérisé en ce que** les branches d'ancrage (2, 3), dans le sens de visualisation par l'avant, sont décalées parallèlement l'une de l'autre de manière à ce que les branches ancrage (2, 3) soient disposées avec la traverse (5) suivant une forme trapézoïdale.
